(19) Europäisches Patentamt
European Patent Office
Office européen des brevets

(11) EP 0 579 059 B1

(12) EUROPEAN PATENT SPECIFICATION

(45) Date of publication and mention of the grant of the patent:
**12.05.1999 Bulletin 1999/19**

(21) Application number: **93110611.6**

(22) Date of filing: **02.07.1993**

(51) Int Cl.[6]: **C07D 237/04**, C07D 237/14, C07D 409/04, A61K 31/50, C07D 401/12, C07D 409/12, C07D 413/12, C07D 417/12, C07D 403/12, C07D 409/06, C07D 401/06, C07D 403/06, C07D 401/14

# (54) Pyridazinone derivatives and processes for preparing the same

Pyridazinone-Derivate und Verfahren zur deren Herstellung

Dérivés de pyridazinone et procédé de préparation

(84) Designated Contracting States:
**AT BE CH DE DK ES FR GB GR IE IT LI LU MC NL PT SE**

(30) Priority: **02.07.1992 JP 215354/92**
**02.07.1992 JP 215355/92**

(43) Date of publication of application:
**19.01.1994 Bulletin 1994/03**

(73) Proprietor: **TANABE SEIYAKU CO., LTD.**
**Chuo-ku Osaka (JP)**

(72) Inventors:
- **Ishida, Akihiko**
  **Urawa-shi, Saitama-ken (JP)**
- **Homma, Koichi**
  **Itabashi-ku, Tokyo-to (JP)**
- **Kono, Harumichi**
  **Omiya-shi, Saitama-ken (JP)**
- **Tamura, Koji**
  **Omiya-shi, Saitama-ken (JP)**
- **Sasaki, Yasuhiko**
  **Urawa-shi, Saitama-ken (JP)**

(74) Representative:
**Hansen, Bernd, Dr. Dipl.-Chem. et al**
**Hoffmann Eitle,**
**Patent- und Rechtsanwälte,**
**Postfach 81 04 20**
**81904 München (DE)**

(56) References cited:
**EP-A- 0 194 548**
**EP-A- 0 253 321**
**EP-A- 0 503 079**
**EP-A- 0 589 037**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates to novel pyridazinone derivatives having actions of protecting from endotoxin shock and actions of curing nephritis, and processes for preparing the same.

In Japanese Provisional Patent Publication No. 23853/1988, it has been disclosed that benzenesulfonamidindanyl compounds such as 6- (2-benzenesulfonamid-indan-5-yl)-4, 5-dihydropyridazin-3(2H)-one exhibit antithrombotic actions.

Further, in Japanese Provisional Patent Publication No. 223277/1991, it has been disclosed that benzothiophene derivatives such as ethyl 4-[4-(4-chlorobenzenesulfonamidemethyl)-4, 5, 6, 7-tetrahydrobenzo [b] thiophen-2-yl]-4-oxo-butanoate exhibit platelet aggregation inhibiting action.

EP-A-0 589 037 which is partly a document in accordance with Art. 54(3) EPC describes an indane derivative or salts thereof which are capable of potently antagonizing the action of thromboxane $A_2$. More specifically, it describes inter alia the preparation of 6-[2-[(4-chlorophenyl) sulfonyl-aminomethyl) indan-5-yl]-3-oxo-2, 3, 4, 5-tetrahydro-pyridazine which may be used for the treatment of nephritis.

EP-O 503 079 which is a document in accordance with Art. 54(3) EPC for a part of the compounds of the present invention reveals pyridazinone-substituted ethynylphenyl derivatives and remedy for circulatory organ disease containing the same as an active ingredient.

On the other hand, as an agent for curing endotoxin shock which occurs in a patient seriously infected with gram-negative bacteria, there have been conventionally used steroid hormones, aprotinin ( a protease inhibitor) and dobutamine (a cardiac).

Further, as an agent for curing nephritis, there have been conventionally used prednisolon, cyclophosphamide, dipyridamole, dilazep and heparin.

An object of the present invention is to provide novel compounds having excellent actions of protecting from endotoxin shock and excellent actions of curing nephritis, and processes for preparing the same.

The pyridazinone derivative according to the present invention is represented by the formula (I):

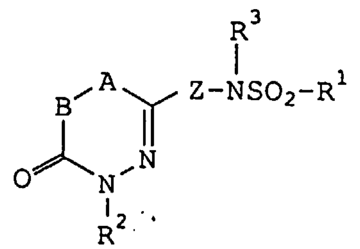

(I)

wherein

$R^1$ represents an alkyl group having 1 to 5 carbon atoms, or an alkenyl group having 2 to 7 carbon atoms,

$R^3$ represents hydrogen atom or an alkyl group having 1 to 6 carbon atoms;

Z represents a group represented by the formula:

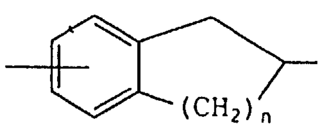

where n represents 1 or 2;

$R^2$ represents hydrogen atom, a carboxyl $C_{1\text{-}6}$ alkyl group a tetrazolyl $C_{1\text{-}6}$ alkyl group or an alkyl group having 1 to 6 carbon atoms;

and -A-B- represents an ethylene group or vinylene group, or a pharmaceutically acceptable salt thereof.

In the desired compound (I) of the present invention, two kinds of optical isomers based on an asymmetric

carbon atom exist. Both of these optical isomers and a mixture thereof are included in the present invention.

The desired compound (I) of the present invention can be formed into a pharmaceutically acceptable salt thereof. As the pharmaceutically acceptable salt, there may be mentioned, for example, inorganic acid salts such as hydrochloride, sulfate and hydrobromide, and organic acid salts such as fumarate, oxalate and maleate.

The desired compound (I) of the present invention can be administered either orally or parenterally, and it can be used as a medical preparation by mixing it with an excipient suitable for oral or parenteral administration. The medical preparation may be a solid preparation such as a tablet, a capsule and a powder, or a liquid preparation such as a solution, a suspension and an emulsion. Further, when the desired compound (I) is administered parenterally, it can be used in the form of an injection.

The dose varies depending on age, weight and state of a patient and disease conditions of a patient, but, in general, the dose per day is preferably 1 to 200 mg/kg, particularly 10 to 100 mg/kg in the case of oral administration, and it is preferably 0.1 to 30 mg/kg, particularly 1 to 20 mg/kg in the case of parenteral administration.

According to the present invention, the desired compound (I) or a pharmaceutically acceptable salt thereof can be prepared by reacting a compound represented by the formula (II):

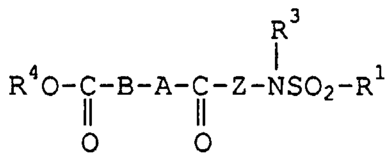

(II)

wherein $R^4$ represents hydrogen atom or a $C_{1-6}$ alkyl group; and $R^1$, $R^3$, Z and -A-B- have the same meanings as defined above,
or a salt thereof, with a compound represented by the formula (III):

$$R^2NH\text{-}NH_2 \quad \text{(III)}$$

wherein $R^2$ has the same meaning as defined above, and, if necessary, converting the resulting compound into a pharmaceutically acceptable salt thereof.

Among the desired compounds (I), a pyridazinone derivative represented by the formula (I-c):

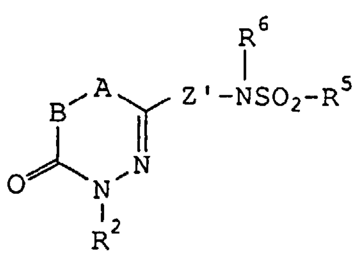

(I-c)

wherein

Z' is a group represented by the formula:

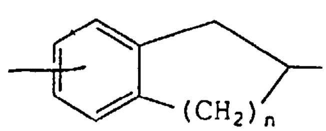

where n has the same meanings as defined above;

$R^5$ represents an alkyl group having 1 to 5 carbon atoms or an alkenyl group having 2 to 7 carbon atoms

$R^6$ represents hydrogen atom or a $C_{1-6}$ alkyl group;

and $R^2$ and -A-B- have the same meanings as defined above,

or a pharmaceutically acceptable salt thereof, can be prepared by reacting a compound represented by the formula (IV) :

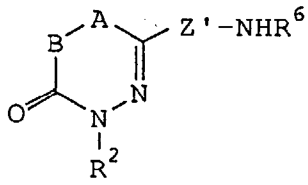

(IV)

wherein $R^2$, $R^6$, Z' and -A-B- have the same meanings as defined above, or a salt thereof, with a compound represented by the formula (V):

$$R^{51}SO_2\text{-}X^1 \quad \text{(V)}$$

wherein $X^1$ represents a reactive residue; and $R^{51}$ represents a substituted or unsubstituted alkyl group having 1 to 5 carbon atoms or an alkenyl group having 2 to 7 carbon atoms, and, if necessary, converting the resulting compound into a pharmaceutically acceptable salt thereof.

Among the desired compounds (I), a pyridazinone derivative represented by the formula (I-e):

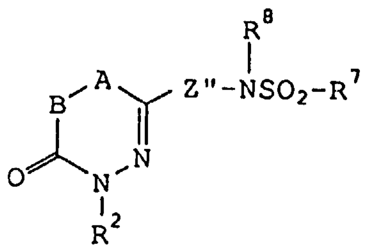

.(I-e)

wherein

$R^7$ represents a $C_{1-5}$ alkyl group or a $C_{2-7}$ alkenyl group,

and Z" represents a group represented by the formula:

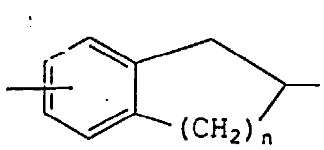

where n has the same meaning as defined above;

$R^8$ represents a $C_{1-6}$-alkyl group and $R^2$ and -A-B- have the same meanings as defined above, or a pharmaceutically acceptable salt thereof, can be prepared by reacting a compound represented by the formula (I-d):

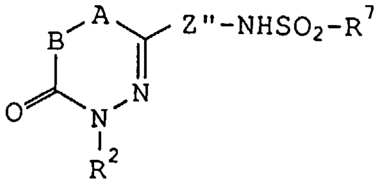

(I-d)

wherein $R^2$, $R^7$, Z" and -A-B- have the same meanings as defined above, or a salt thereof, with a compound represented by the formula (VI):

$$R^8\text{-}X^2 \qquad (VI)$$

wherein $X^2$ represents a reactive residue and $R^8$ has the same meaning as defined above, and, if necessary, converting the resulting compound into a pharmaceutically acceptable salt thereof.

Among the desired compounds (I), a pyridazinone derivative represented by the formula (I-g):

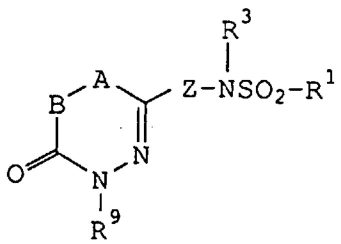

(I-g)

wherein $R^9$ represents a carboxyl $C_{1\text{-}6}$ alkyl group, a tetrazolyl $C_{1\text{-}6}$ alkyl group, or an alkyl group having 1 to 6 carbon atoms; and $R^1$, $R^3$, Z and -A-B- have the same meanings as defined above, or a pharmaceutically acceptable salt thereof, can be prepared by reacting a compound represented by the formula (I-f):

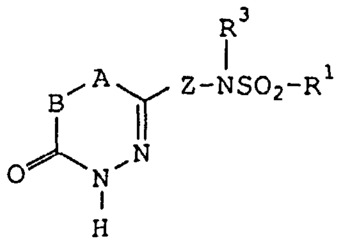

(I-f)

wherein $R^1$, $R^3$, Z and -A-B- have the same meanings as defined above, or a salt thereof, with a compound represented by the formula (VII):

$$R^9\text{-}X^3 \qquad (VII)$$

wherein $X^3$ represents a reactive residue; and $R^9$ has the same meaning as defined above, and, if necessary, converting the resulting compound into a pharmaceutically acceptable salt thereof.

Among the desired compounds (I), a pyridazinone derivative represented by the formula (I-i):

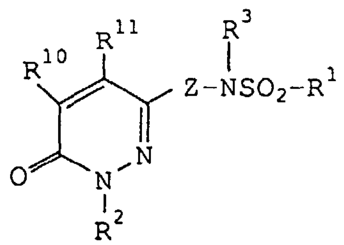

(I-i)

wherein $R^{10}$ and $R^{11}$ represent hydrogen atom, and $R^1$, $R^2$, $R^3$ and Z have the same meanings as defined above, or a pharmaceutically acceptable salt thereof, can be prepared by oxidizing a compound represented by the formula (I-h):

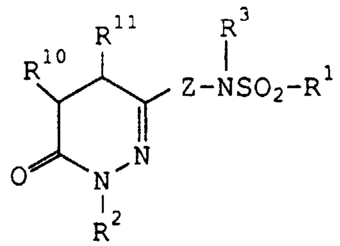

(I-h)

wherein $R^1$, $R^2$, $R^3$, $R^{10}$, $R^{11}$ and Z have the same meanings as defined above,
or a salt thereof, and, if necessary, converting the resulting compound into a pharmaceutically acceptable salt thereof.

In the above preparation process of the present invention, the reaction of the compound (II) and the hydrazine compound (III) can be carried out suitably in the presence or absence of a suitable solvent.

The solvent may be any inactive solvent which does not exert bad influence on the reaction, and may include, for example, a lower alcohol, a lower aliphatic acid such as acetic acid and propionic acid, an aromatic hydrocarbon such as toluene and xylene, and an ether such as tetrahydrofuran and dioxane.

The hydrazine compound (III) may be a hydrate.

The reaction can be carried out at a wide range of temperature from room temperature to a boiling point of a reaction mixture, for example, preferably 10 °C to 200 °C, particularly 20 °C to 150 °C.

The condensation reaction of the compound (IV) and the compound (V) can be carried out suitably in a suitable solvent in the presence or absence of an acid acceptor, respectively.

As the reactive residue ($X^1$) of the compound (V), a group which splits off nucleophilically, for example, a halogen atom or an alkoxy group may be suitably used, or when the compound (V) is a symmetric sulfonic anhydride, a lower alkylsulfonyloxy group, benzenesulfonyloxy group or a lower alkyl group-substituted benzenesulfonyloxy group may be suitably used.

As the acid acceptor, there may be used suitably, for example, organic bases such as a tri-lower alkylamine, an N-lower alkylmorpholine, pyridine and 2,6-lutidine, and inorganic bases such as an alkali metal hydroxide, an alkali metal hydrogen carbonate, an alkali metal carbonate and an alkali metal hydride.

The solvent may be any innert solvent which does not exert bad influence on the reaction, and may include, for example, halogen type solvents such as chloroform, dichloromethane and dichloroethane; aromatic hydrocarbons such as toluene and xylene; ethers such as tetrahydrofuran and dioxane; ketone type solvents such as acetone and methyl ethyl ketone; esters such as ethyl acetate; acetonitrile; pyridine; 2,6-lutidine; dimethylformamide; dimethylsulfoxide; 1,3-dimethyl-2-imidazolidinone; and a combination of these solvents and water.

The condensation reaction can be carried out at a wide range of temperature from -78 °C to a boiling point of a reaction mixture, for example, preferably -10 °C to 150 °C, particularly -10 °C to 80 °C.

The condensation reactions of the compound (I-d) and the compound (VI), and the compound (I-f) and the compound (VII) can be carried out suitably in a suitable solvent in the presence of a base, respectively.

As the reactive residues ($X^2$ and $X^3$) of the compounds (VI) and (VII), a group which splits off nucleophilically, for example, a halogen atom, an alkoxy group, a lower alkylsulfonyloxy group, a benzenesulfonyloxy group, a lower alkyl-substituted benzenesulfonyloxy group and a trifluoromethanesulfonyloxy group may be suitably used.

As the base, there may be used suitably, for example, lower alkyl metal such as n-butyl lithium, alkali metal alkoxide, alkali metal hydroxide, alkali metal carbonate and alkali metal hydride.

The solvent may be any innert solvent which does not exert bad influence on the reaction, and may include,

for example, ethers such as dimethoxyethane, tetrahydrofuran and dioxane; alcohols such as methanol, ethanol, 2-methoxyethanol, propanol and butanol; ketones such as acetone and methyl ethyl ketone; esters such as ethyl acetate; amide type solvents such as dimethylformamide, dimethylacetamide and 1,3-dimethyl-2-imidazolidinone; acetonitrile; and dimethylsulfoxide.

The condensation reaction can be carried out under cooling or with heating, particularly 0 °C to 150 °C.

The oxidation of the compound (I-h) can be carried out according to a conventional method, and it can be carried out preferably by, for example, treating the compound (I-h) with sodium 3-nitrobenzenesulfonate in a suitable solvent under basic conditions; subjecting it to oxidation by using dimethylsulfoxide under acidic conditions; or treating it with hydrogen bromide-acetic acid or the like.

As the solvent, there may be used suitably water, acetic acid, trifluoroacetic acid, methanesulfonic acid and an acetic acid solution of hydrogen bromide.

In the reactions of the present invention, by using an optical isomer as the starting compound (II), (IV), (I-d), (I-f) or (I-h), a corresponding optically active desired compound (I), (I-c), (I-e), (I-g) or (I-i) can be obtained, respectively, without racemization.

The starting compound (II) can be prepared by, for example, reacting a compound represented by the formula (X):

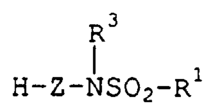

(X)

wherein $R^1$, $R^3$ and Z have the same meanings as defined above,
which can be obtained by reacting a compound represented by the formula (VIII):

$$H\text{-}Z'\text{-}NHR^6 \qquad (VIII)$$

wherein $R^6$ and Z' have the same meanings as defined above,
with the sulfone compound (V) or a compound represented by the formula (IX):

$$X^5\text{-}SO_2\text{-}E\text{-}X^4 \qquad (IX)$$

wherein E represents a lower alkylene group; and $X^4$ and $X^5$ each represent a reactive residue,
and, if necessary, further carrying out cyclization in the molecule, with a compound represented by the formula (XI):

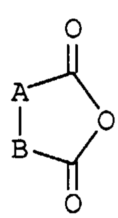

(XI)

wherein -A-B- has the same meaning as defined above, or a compound represented by the formula (XII):

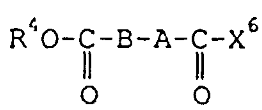

(XII)

wherein $X^6$ represents a reactive residue; and $R^4$ and -A-B- have the same meanings as defined above.

The starting compound (IV) can be prepared by, for example, reacting a compound represented by the formula (XIII):

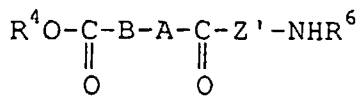

(XIII)

wherein $R^4$, $R^6$, Z' and -A-B- have the same meanings as defined above,
with the hydrazine compound (III), and when -A-B- is ethylene group, further oxidizing the reaction product to convert said ethylene group into vinylene group, if desired.

The starting compound (XIII) of the above reaction can be prepared by, for example, a method in which, if necessary, after an amino group of the compound (VIII) is protected, said compound is reacted with the compound (XI) or (XII), and then the protective group is removed, or a method described below in Examples described below.

In the present specification, the lower alkyl group, lower alkoxy group and lower alkylene group include those having 1 to 6 carbon atoms, and the lower alkenyl group includes those having 2 to 7 carbon atoms.

The present invention is described in detail by referring to Examples.

Test example 1

(Action of protecting mouse from death induced by endotoxin)

To ddy strain male mice which had fasted for about 24 hours, a test sample, 2-[4,5-dihydropyridazin-3(2H)-on-6-yl]-5-(n-butylsulfonylamino)-4,5,6,7-tetrahydrobenzo[b]thiophene dissolved or suspended in a 0.25 % sodium carboxymethyl cellulose (CMC) aqueous solution was orally administered (100 mg/kg). After 30 minutes, 100 mg/10 ml/kg of endotoxin (lipopolysaccharide) derived from *Escherichia coli* dissolved in physiological saline was administered intraperitoneally. When the survival rate of the control group to which the CMC aqueous solution had been orally administered became 20 % (about 20 hours after administration of endotoxin), the survival rate of the group to which the test sample had been administered was determined. As a result, the survival rate of the group to which the test sample had been administered was 90 %.

Test example 2

(Action of protecting mouse from death induced by endotoxin)

To ddy strain male mice which had fasted for about 24 hours, a test sample, 2-methylsulfonylamino-5-[4,5-dihydropyridazin-3(2H)-on-6-yl]indane dissolved or suspended in a 0.25 % sodium carboxymethyl cellulose (CMC) aqueous solution was orally administered (100 mg/kg). After 30 minutes, 100 mg/10 ml/kg of endotoxin (lipopolysaccharide) derived from *Escherichia coli* dissolved in physiological saline was administered intraperitoneally. When the survival rate of the control group to which the CMC aqueous solution had been orally administered became 20 % (about 20 hours after administration of endotoxin), the survival rate of the group to which the test sample had been administered was determined. As a result, the survival rate of the group to which the test sample had been administered was 100 %.

Test example 3

(Action of curing nephritis)

To male WKY rats (8 weeks old, 6 rats per group), 2.5 ml/kg of a rabbit anti-glomerular basement membrane serum diluted by 50 times with physiological saline was administered intravenously to induce nephritis. A test sample, 2-propylsulfonylamino-5-[pyridazin-3(2H)-on-6-yl]indane suspended in purified water by using Tween 80 (trade name, produced by Nacalai Tesque Co.) was orally administered in a dose of 30 mg/kg/10 ml twice a day for 8 days. Eight days after induction of nephritis, urine was collected for 24 hours. By measuring urine volumes and measuring concentrations of protein in urine by the sulfosalicylic acid method, amounts of protein excreted in urine were calculated. The protein excretion of the group which had been administered the test sample was decreased to 50 to 55 % protein excretion of the control group.

Example 1

1.4 g of potassium carbonate dissolved in 20 ml of water was added to 1.15 g of 2-amino-5-[4,5-dihydropyridazin-3(2H)-on-6-yl]indane dissolved in 40 ml of ethyl acetate and 20 ml of tetrahydrofuran. Then, 0.57 g of methanesulfonyl chloride was added thereto and the mixture was stirred for 2 hours. After the organic layer was collected

by separation and dried, the solvent was removed. The resulting crystals were recrystallized from tetrahydrofuran-isopropyl ether to obtain 1.08 g of 2-methylsulfonylamino-5-[4,5-dihydropyridazin-3(2H)-on-6-yl]indane.
m.p.: 218 to 220 °C.

Example 2

6.3 g of n-butanesulfonyl chloride was added under ice cooling to 5.61 g of 2-amino-5-[4,5-dihydropyridazin-3(2H)-on-6-yl]indane and 4.1 g of triethylamine suspended in 50 ml of chloroform, and the mixture was stirred for 1 hour. Diluted hydrochloric acid was added to the reaction mixture, the chloroform layer was collected by separation, washed and dried, and then the solvent was removed. The resulting crystals were recrystallized from tetrahydrofuran-isopropyl ether to obtain 6.40 g of 2-n-butylsulfonylamino-5-[4,5-dihydropyridazin-3(2H)-on-6-yl]indane.
m.p.: 183 to 185 °C.

Examples 3 to 28

By treating the corresponding starting compounds in the same manner as in Example 2, compounds shown in the following Table 1 were obtained.

Table 1

| Example No. | 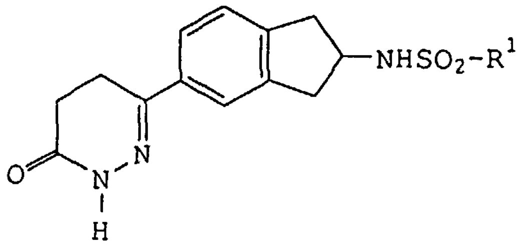 | |
|---|---|---|
| | $R^1$ | Physical properties |
| 3 | $-C_2H_5$ | m.p. 177 - 178 °C |
| 4 | $-n-C_3H_7$ | m.p. 178 - 179 °C |
| 5 | $-CH_2CH(CH_3)_2$ | m.p. 193.5 - 194.5 °C |
| 6 | $-n-C_5H_{11}$ | m.p. 175.5 - 176.5 °C |
| 7 | $-n-C_6H_{13}$ | m.p. 173 - 174 °C |
| 8 | $-n-C_7H_{15}$ | m.p. 174 - 175 °C |
| 9 | $-n-C_8H_{17}$ | m.p. 193 - 195 °C |
| 10 | $-CH(CH_3)_2$ | m.p. 160 - 162 °C |

Example 11

(1) In 1,400 ml of ethanol was dissolved 8.41 g of 2-amino-5-[4,5-dihydropyridazin-3 (2H)-on-6-yl]indane, and 8.52 g of (+)-camphorsulfonic acid was added thereto. The resulting salts were recrystallized from ethanol several times to obtain 5.20 g of optically active 2-amino-5-[4,5-dihydropyridazin-3 (2H)-on-6-yl]indane·(+)-camphorsulfonate.
m.p.: 256 to 257 °C (decomposed)
$[\alpha]_D^{20}$ : -13.4° (c = 1.00, $H_2O$).

(2) The product obtained was treated in the same manner as in Example 2 to obtain (+)-2-n-butylsulfonylamino-5-[4,5-dihydrppyridazin-3(2H)-on-6-yl]indane.
m.p.: 173 to 174 °C
$[\alpha]_D^{20}$ : +33>2° (c = 1.00, dimethylformamide (DMF)).

Example 12

(1) All mother liquor of Example 11-(1) was recovered and neutralized with a potassium carbonate aqueous solution, and ethanol was removed. The residue was extracted with ethyl acetate, and ethyl acetate was removed. In 1,000 ml of ethanol was dissolved 5.23 g of the residue, and 5.29 g of (-)-camphorsulfonic acid was added thereto. The resulting salts were recrystallized from ethanol several times to obtain 4.99 g of optically active 2-amino-5-[4,5-dihydropyridazin-3(2H)-on-6-yl]indane (-)-camphorsulfonate.
m.p.: 257 to 258 °C (decomposed,
$[\alpha]_D^{20}$ : +13.1° (c =1.00, $H_2O$).

(2) The product obtained was treated in the same manner as in Example 2 to obtain (-)-2-n-butylsulfonylamino-5-[4,5-dihydropyridazin-3(2H)-on-6-yl]indane.
m.p.: 173 to 174 °C
$[\alpha]_D^{20}$ : -33.4° (c = 1.00, DMF).

Example 13

(1) To 87.44 g of 2-aminoindane dissolved in 800 ml of methylene chloride was added 66.43 g of triethylamine, and 102.8 g of n-butylsulfonyl chloride was added dropwise thereto under ice cooling. The mixture was stirred at the same temperature for 1 hour. The reaction mixture was washed with diluted hydrochloric acid and dried, and then the solvent was removed. The resulting crystals were recrystallized from isopropyl ether to obtain 151.12 g of 2-n-butylsulfonylaminoindane.
m.p.: 60 to 62 °C.

(2) To 29.22 g of the product obtained and 23.05 g of succinic anhydride suspended in 300 ml of dichloroethane was added 62.96 g of anhydrous aluminum chloride under ice cooling, and the mixture was stirred at room temperature for 1 hour. To the reaction mixture were added 200 ml of 10 % hydrochloric acid under ice cooling and then 700 ml of ethyl acetate. After the organic layer was collected by separation, washed and dried, the solvent was removed. The resulting crystals were recrystallized from ethyl acetate to obtain 29.77 g of 2-n-butylsulfonylamino-5-(3-carboxypropionyl)indane.
m.p.: 138.5 to 139.5 °C.

(3) In 200 ml of ethanol was dissolved 17.30 g of the product obtained, and 12.5 g of hydrazine monohydrate was added thereto. The mixture was refluxed under heating for 2.5 hours. The solvent was removed from the reaction mixture, and the residue was washed with water, dried and recrystallized from tetrahydrofuran-isopropyl ether to obtain 12.04 g of 2-n-butylsulfonylamino-5-[4,5-dihydropyridazin-3(2H)-on-6-yl]indane.
m.p.: 183 to 185 °C.

Example 14

By reacting 2-n-butylsulfonylamino-5-(3-carboxypropionyl)indane with substituted hydrazine in the same manner as in Example 13, the compound shown in the following Table 2 was obtained.

Table 2

| Example No. | 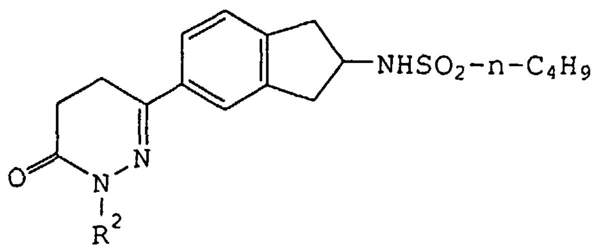 | |
|---|---|---|
| | $R^2$ | Physical properties |
| 14 | $-CH_3$ | m.p. 119 - 122 °C |

Example 15

(1) In 100 ml of tetrahydrofuran was suspended 5.7 g of lithium aluminum hydride, and to the suspension was added dropwise 18.9 g of 2-propionylaminoindane dissolved in 150 ml of tetrahydrofuran. The mixture was refluxed under heating for 2 hours. After cooling, excessive lithium aluminum hydride was treated with a saturated ammonium chloride aqueous solution, and insolubles were removed by filtration. The filtrate was condensed, and chloroform was added to the residue. After the mixture was washed with water and dried, the solvent was removed to obtain 17.5 g of 2-propylaminoindane.

In 200 ml of tetrahydrofuran were dissolved 17.5 g of the product obtained and 12.12 g of triethylamine, and to the solution was added dropwise 17.8 g of methyl chlorocarbonate under cooling. The mixture was stirred at room temperature for 2 hours, tetrahydrofuran was removed, and ethyl acetate was added to the residue. After the mixture was washed with water and dried, the solvent was removed to obtain 21.6 g of 2-(N-methoxycarbonyl-N-propyl)aminoindane.

$IR^{neat}$ $\nu_{max}$ ($cm^{-1}$): 1660

Mass (m/z): 233 ($M^+$).

(2) To 21.6 g of the product obtained and methylsuccinyl chloride (prepared from 24.68 g of methyl hydrogen succinate and 23.75 g of oxalyl chloride) dissolved in 300 ml of dichloromethane was added 59.85 g of anhydrous aluminum chloride under ice cooling, and the mixture was stirred at room temperature for 2 hours. The reaction mixture was poured into ice water, the organic layer was collected by separation, washed with water and dried, and then the solvent was removed to obtain 33.72 g of 2-(N-methoxycarbonyl-N-propyl)amino-5-(3-methoxycarbonylpropionyl)indane.

To 33.72 g of the product obtained dissolved in xylene were added 12.75 g of hydrazine monohydrate and 24 ml of acetic acid, and the mixture was refluxed under heating for 4 hours. The reaction mixture was poured into ice water and extracted with ethyl acetate, the extract was washed with water and dried, and then the solvent was removed. The residue was solidified in a mixed solution of diisopropyl ether and hexane to obtain 28.96 g of 2-(N-methoxycarbonyl-N-propyl)amino-5-[4,5-dihydropyridazin-3(2H)-on-6-yl]indane.

$IR^{neat}$ $\nu_{max}$ ($cm^{-1}$): 3200, 1680, 1660

Mass (m/z): 329 ($M^+$).

(3) To 13.16 g of the product obtained dissolved in 130 ml of chloroform was added 9 ml of iodotrimethylsilane, and the mixture was refluxed under heating for 2 hours. After cooling, aqueous ammonia was added to the mixture, the organic layer was collected by separation, washed with water and dried, and then the solvent was removed. The resulting crude crystals were recrystallized from acetone to obtain 7.78 g of 2-propylamino-5-[4,5-dihydropyridazin-3(2H)-on-6-yl]indane.
m.p.: 146 to 148 °C.

(4) The product obtained and n-butylsulfonyl chloride were treated in the same manner as in Example 2 to obtain 28.96 g of 2-(N-n-butylsulfonyl-N-propyl)amino-5-[4,5-dihydropyridazin-3(2H)-on-6-yl]indane.
m.p.: 137 to 139 °C.

Example 16

2-Amino-5-[4,5-dihydropyridazin-3(2H)-on-6-yl]indane was treated with chloroethanesulfonyl chloride in the presence of two equivalent amounts of triethylamine in the same manner as in Example 2 to obtain 2-vinylsulfonylamino-5-[4,5-dihydropyridazin-3(2H)-on-6-yl]indane.
m.p.: 168 to 170 °C.

Example 17

To 1.0 g of 2-n-butylsulfonylamino-5-[4,5-dihydropyridazin-3(2H)-on-6-yl]indane dissolved in 15 ml of dimethylformamide was added 118 mg of 62.6 % sodium hydride under ice cooling, and the mixture was stirred for 20 minutes. To the mixture was added 544 mg of propyl iodide dissolved in 5 ml of dimethylformamide, and the mixture was stirred at room temperature overnight. The reaction mixture was poured into ice water and extracted with ethyl acetate, the extract was washed with water and dried, and then the solvent was removed. The residue was purified by silica gel column chromatography (eluted by chloroform:ethyl acetate = 5:1) to obtain 690 mg of 2-(N-n-butylsulfonyl-N-propyl)amino-5-[4,5-dihydropyridazin-3(2H)-on-6-yl]indane. This compound had the same physical property values as those of the compound obtained in Example 15.

Examples 18 to 21

By treating the corresponding starting compounds in the same manner as in Example 17, compounds shown in the following Table 3 were obtained.

Table 3

| Example No. | 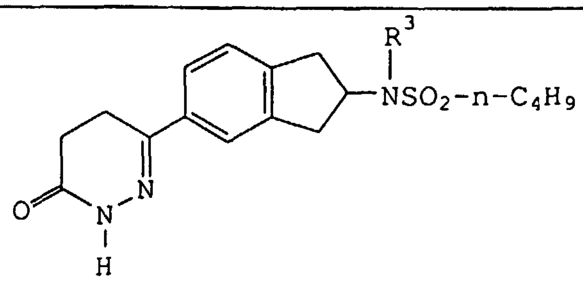 | |
|---|---|---|
| | $R^3$ | Physical properties |
| 18 | $-CH_3$ | m.p. 152 - 154 °C |
| 19 | $-CH_2CH_3$ | m.p. 125 - 126 °C |
| 20 | $-CH_2CH(CH_3)_2$ | m.p. 138 - 140 °C |
| 21 | $-(CH_2)_3CH_3$ | MS (m/z): 405 ($M^+$)<br>IR (nujol) $cm^{-1}$: 3270, 1680, 1630 |

Example 22

2-n-Butylsulfonylamino-5-[2-methyl-4,5-dihydropyridazin-3(2H)-on-6-yl]indane and methyl iodide were treated in the same manner as in Example 17 to obtain 2-(N-n-butylsulfonyl-N-methyl)amino-5-[2-methyl-4,5-dihydropyridazin-3(2H)-on-6-yl]indane.
m.p.: 137 to 138 °C.

Example 23

2-n-Butylsulfonylamino-5-{2-[3-(1-imidazolyl)propyl]pyridazin-3(2H)-on-6-yl}indane and propyl iodide were treated in the same manner as in Example 17 to obtain 2-(N-n-butylsulfonyl-N-propyl)amino-5-{2-[3-(1-imidazolyl)propyl]pyridazin-3(2H)-on-6-yl}indane.
$IR^{neat}$ $\nu_{max}$ ($cm^{-1}$): 1670
FAB-MS (m/z): 498 ($MH^+$).

Example 24

(1) In 70 ml of a 25 % hydrogen bromide-acetic acid solution was suspended 8.02 g of 2-amino-5-[4,5-dihydropyridazin-3(2H)-on-6-yl]indane, and 2.96 g of dimethylsulfoxide was added thereto under ice cooling. The mixture was stirred at room temperature for 1.5 hours. Then, 140 ml of diethyl ether was added to the reaction mixture, and crystals precipitated were collected by filtration and recrystallized from methanol to obtain 8.55 g of 2-amino-5-[pyridazin-3(2H)-on-6-yl]indane·hydrobromide.
m.p.: >300 °C.

The product obtained was treated with sodium hydroxide to obtain 2-amino-5-[pyridazin-3(2H)-on-6-yl]indane.
m.p.: 228 to 229 °C.

(2) In 50 ml of dimethylformamide were dissolved 1.14 g of 2-amino-5-[pyridazin-3(2H)-on-6-yl]indane and 0.8 g of triethylamine, and 1.25 g of n-butanesulfonyl chloride was added thereto. The mixture was stirred at 50 °C for 30 minutes and then stirred at room temperature overnight. The solvent was removed, water was added to the residue, and crystals precipitated were collected by filtration. The resulting crystals were recrystallized from methanol to obtain 1.27 g of 2-n-butylsulfonylamino-5-[pyridazin-3(2H)-on-6-yl]indane.
m.p.: 195 to 196 °C.

Example 25

(1) 2-Propylamino-5-[4,5-dihydropyridazin-3(2H)-on-6-yl]indane was treated in the same manner as in Example 24-(1) to obtain 2-propylamino-5- [pyridazin-3 (2H)-on-6-yl]-indane.
m.p.: 137 to 139 °C.

(2) The product obtained and n-butylsulfonyl chloride were treated in the same manner as in Example 2 to obtain 2-(N-n-butylsulfonyl-N-propyl)amino-5-[pyridazin-3(2H)-on-6-yl]indane.
m.p.: 142 to 143 °C.

Example 26

2-Propylamino-5-[pyridazin-3(2H)--on-6-yl]indane and chloroethanesulfonyl chloride were treated in the presence of two equivalent amounts of triethylamine in the same manner as in Example 2 to obtain 2-(N-vinylsulfonyl-N-propyl)amino-5[pyridazin-3(2H)-on-6-yl]indane.
m.p.: 153 to 154 °C.

Example 27

(1) In 100 ml of a 25 % hydrogen bromide-acetic acid solution was suspended 17.44 g of 2-n-butylsulfonylamino-5-[4,5-dihydropyridazin-3(2H)-on-6-yl]indane, and 3.6 ml of dimethylsulfoxide was added thereto. The mixture was stirred at room temperature for 1 hour. Then, the reaction mixture was poured into ice water, and crystals precipitated were collected by filtration and recrystallized from methanol to obtain 14.60 g of 2-n-butylsulfonylamino-5-[pyridazin-3(2H)-on-6-yl]indane.
m.p.: 195 to 196 °C.

Examples 28 to 39

By treating the corresponding starting compounds in the same manner as in Example 27, compounds shown in the following Tables 4 to 6 were obtained.

Table 4

| Example No. | 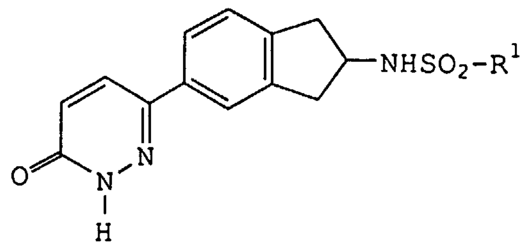 | |
|---|---|---|
| | $R^1$ | Physical properties |
| 28 | $-CH_3$ | m.p. 228 - 229 °C |
| 29 | $-n-C_3H_7$ | m.p. 199 - 200 °C |
| 30 | $-CH(CH_3)_2$ | m.p. 213 - 215 °C |
| 31 | $-CH_2CH(CH_3)_2$ | m.p. 218 - 219 °C |
| 32 | $-n-C_5H_{11}$ | m.p. 201 - 202 °C |
| 33 | $-n-C_6H_{13}$ | m.p. 190 - 192 °C |
| 34 | $-n-C_7H_{15}$ | m.p. 186 - 187 °C |

Table 4 (cont'd)

| Example No. | 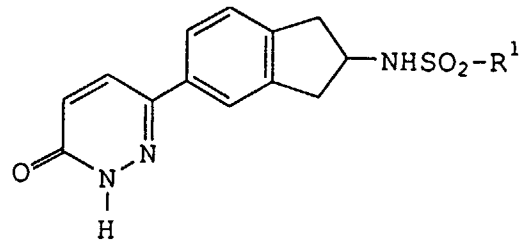 | |
|---|---|---|
| | $R^1$ | Physical properties |
| 35 | $-CH=CH_2$ | m.p. 200 - 202 °C |
| 36 | $-CH_2CH_3$ | m.p. 220 - 221 °C |

Table 5

| Example No. | 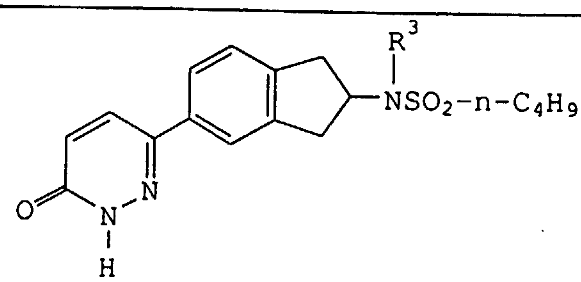 | |
|---|---|---|
| | $R^3$ | Physical properties |
| 37 | $-CH_3$ | m.p. 168 - 169 °C |
| 38 | $-n-C_3H_7$ | m.p. 142 - 143 °C |

Table 6

| Example No. | 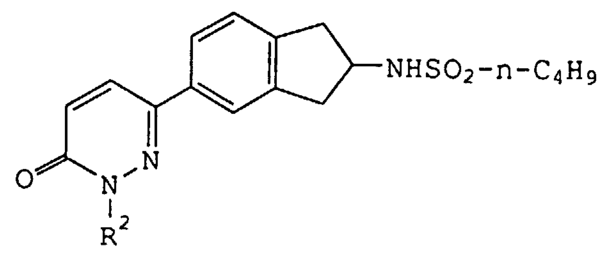 | |
|---|---|---|
| | $R^2$ | Physical properties |
| 39 | $-CH_3$ | m.p. 152 - 153 °C |

Example 40

In 55 ml of water were dissolved 2.45 g of 2-n-butylsulfonylamino-5-[4,5-dihydropyridazin-3(2H)-on-6-yl]indane and 1.40 g of sodium hydroxide, and 2.66 g of sodium 3-nitrobenzenesulfonate was added thereto. The mixture was refluxed under heating for 5 hours. After cooling, the reaction mixture was made acidic with 10 % hydrochloric acid and extracted with ethyl acetate, the extract was dried, and then the solvent was removed. The resulting crystals were recrystallized from methanol to obtain 1.62 g of 2-n-butylsulfonylamino-5-[pyridazin-3(2H)-on-6-yl]indane.
m.p.: 195 to 196 °C.

Example 41

11.3 ml of a 1N sodium hydroxide aqueous solution was added dropwise under ice cooling to 3.25 g of 2-n-butylsulfonylamino-5-[2-ethoxycarbonylmethylpyridazin-3(2H)-on-6-yl]indane suspended in 50 ml of methanol, and the mixture was stirred at room temperature for 5 hours. Methanol was removed, water was added to the residue, and the mixture was made acidic with 10 % hydrochloric acid. Crystals precipitated were collected by filtration, washed with water and then recrystallized from ethanol-water to obtain 2.50 g of 2-n-butylsulfonylamino-5-[2-carboxymethylpyridazin-3(2H)-on-6-yl]indane.
m.p.: 186 to 188 °C.

Example 42

To 2.23 g of 2-n-butylsulfonylamino-5-[2-cyanomethylpyridazin-3(2H)-on-6-yl]indane dissolved in 45 ml of dimethylformamide were added 1.8 g of sodium azide and 1.5 g of ammonium chloride, and the mixture was stirred at 95 °C for 3.5 hours. After cooling, water was added to the reaction mixture, and the mixture was made acidic with 10 % hydrochloric acid. Crystals precipitated were collected by filtration, dried and then recrystallized from methanol to obtain 2.19 g of 2-n-butylsulfonylamino-5-[2-(5-tetrazolyl)methylpyridazin-3(2H)-on-6-yl]indane.
m.p.: 192 to 194 °C.

Example 43

To 10.3 g of 2-amino-6-[4,5-dihydropyridazin-3(2H)-on-6-yl]-1,2,3,4-tetrahydronaphthalene·hydroiodide suspended in 100 ml of 1,3-dimethyl-2-imidazolidinone was added 26.7 ml of triethylamine at room temperature, and the mixture was stirred for 30 minutes. Under ice cooling, to the mixture was added dropwise 12.6 ml of butanesulfonyl chloride, and the mixture was stirred at room temperature for 4 hours. To the mixture was added 40 ml of ethanol, and the mixture was further stirred for 30 minutes. Ethyl acetate was added to the reaction mixture, the organic layer was washed with water and dried, and then the solvent was removed. The resulting crude crystals were recrystallized from isopropanol to obtain 8.5 g of 2-n-butylsulfonylamino-6-[4,5-dihydropyridazin-3(2H)-on-6-yl]-1,2,3,4-tetrahydronaphthalene.
m.p.: 185 to 187 °C.

Example 44

By treating the corresponding starting compounds in the same manner as in Example 43, the compound shown in the following Table 9 was obtained.

Table 9

| Example No. | 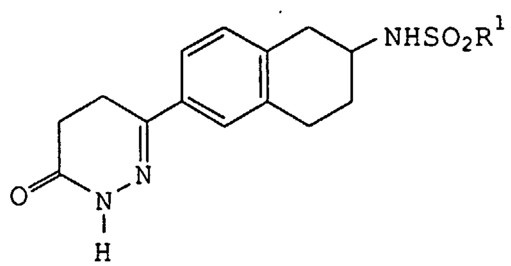 | |
|---|---|---|
| | $R^1$ | Physical properties |
| 44 | $-C_2H_5$ | m.p. 157 - 158 °C |

Example 45

2-n-Butylsulfonylamino-6-[4,5-dihydropyridazin-3(2H)-on-6-yl]-1,2,3,4-tetrahydronaphthalene was treated with dimethylsulfoxide-25 % hydrogen bromide-acetic acid in the same manner as in Example 27 to obtain 2-n-butylsulfonylamino-6-[pyridazin-3(2H)-on-6-yl]-1,2,3,4-tetrahydronaphthalene.
m.p.: 195 to 197 °C.

Example 46

2-n-Propylsulfonylamino-5-[pyridazin-3(2H)-on-6-yl]indane and methyl iodide were reacted to obtain 2-n-propylsulfonylamino-5-[2-methylpyridazin-3(2H)--on-6-yl]indane.
m.p.: 182 to 183 °C.

Reference example 1

(1) To 17.58 g of 2-methoxycarbonylaminosuccinic anhydride and 33.77 g of 4-chlorobenzene suspended in 150 ml of dichloroethane was added 33.40 g of anhydrous aluminum chloride under ice cooling, and the mixture was stirred at room temperature for 1 hour. Under ice cooling, to the mixture were added 100 ml of 10 % hydrochloric acid and then 500 ml of ethyl acetate. After the organic layer was collected by separation, washed with water and dried, the solvent was removed. The resulting crude crystals were recrystallized from ethyl acetate to obtain 24.2 g of 3-carboxy-3-methoxycarbonylamino-4'-chloropropiophenone.
m.p.: 141 to 142 °C.

(2) To 45.57 g of the above compound and 16.97 g of triethylamine dissolved in 400 ml of tetrahydrofuran was added dropwise 18.20 g of trimethylchlorosilane under ice cooling, and the mixture was stirred at room temperature for 1.5 hours. Triethylamine hydrochloride formed was removed, and tetrahydrofuran was removed. The residue was dissolved in 400 ml of dichloromethane, and 55.67 g of triethylsilane was added thereto. Then, to the mixture

was added dropwise 90.9 g of titanium tetrachloride under ice cooling, and the mixture was stirred at room temperature for 18 hours. The reaction mixture was poured into conc. hydrochloric acid (50 ml)-ice and extracted with 1,000 ml of ethyl acetate. After the organic layer was collected by separation, washed with water and dried, the solvent was removed. The residue was purified by silica gel column chromatography (eluted by chloroform:methanol = 100:1) to obtain 39.68 g of 1-chloro-4-(3-carboxy-3-methoxycarbonylaminopropyl)benzene.
m.p.: 113 to 114 °C.

(3) To 10.0 g of the above compound dissolved in 200 ml of dichloromethane were added 3.9 ml of oxalyl chloride and one drop of dimethylformamide, and the mixture was stirred at room temperature for 30 minutes. To the mixture was added 12.4 g of anhydrous aluminum chloride under ice cooling, and the mixture was stirred at room temperature for 30 minutes. The reaction mixture was poured into ice water and extracted with ethyl acetate (1,000 ml). After the organic layer was washed with water and dried, the solvent was removed. The resulting crude crystals were recrystallized from a mixed solution of ethyl acetate and hexane to obtain 7.30 g of 1-oxo-2-methoxycarbonylamino-7-chloro-1,2,3,4-tetrahydronaphthalene.
m.p.: 126 to 127 °C.

(4) To 13.47 g of the above compound dissolved in 40 ml of trifluoroacetic acid was added 24.1 g of triethylsilane. After the mixture was refluxed under heating for 3 hours, trifluoroacetic acid was removed. The residue was dissolved in ethyl acetate. After the solution was washed with water and dried, the solvent was removed. The resulting crude crystals were recrystallized from a mixed solution of ethyl acetate and hexane to obtain 10.52 g of 2-methoxycarbonylamino-7-chloro-1,2,3,4-tetrahydronaphthalene.
m.p.: 99 to 101 °C.

(5) To 14.9 g of methyl hydrogen succinate dissolved in 180 ml of dichloromethane were added 10.3 ml of oxalyl chloride and one drop of dimethylformamide, and the mixture was stirred at room temperature for 3 hours. To the mixture were added 18.0 g of 2-methoxycarbonylamino-7-chloro-1,2,3,4-tetrahydronaphthalene obtained above and 40.0 g of anhydrous aluminum chloride under ice cooling, and the mixture was stirred at room temperature for 18 hours. The reaction mixture was poured into ice water, and the organic layer was collected by separation. After the organic layer was washed with water and dried, the solvent was removed. The resulting crude crystals were recrystallized from a mixed solution of ethyl acetate and hexane to obtain 24.3 g of 2-methoxycarbonylamino-6-(3-methoxycarbonylpropionyl)-7-chloro-1, 2,3,4-tetrahydronaphthalene.
m.p.: 86 to 89 °C.

(6) To 30.0 g of the above compound dissolved in 600 ml of dimethylformamide were added 60 ml of 90 % formic acid and 7.5 g of 10 % palladium-carbon, and the mixture was refluxed under heating for 15 hours. The residue was dissolved in ethyl acetate. After the solution was washed with water and dried, the solvent was removed to obtain 26.0 g of 2-methoxycarbonylamino-6-(3-methoxycarbonylpropionyl)-1,2,3,4-tetrahydronaphthalene.
m.p.: 109 to 111 °C.

(7) To 26.0 g of the above compound dissolved in 260 ml of xylene were added 20.0 g of hydrazine monohydrate and 23 ml of acetic acid, and the mixture was refluxed under heating for 4 hours. Xylene was removed, and the resulting crude crystals were washed with water, dried and then recrystallized from isopropanol-diisopropyl ether to obtain 20.0 g of 2-methoxycarbonylamino-6-[4,5-dihydropyridazin-3(2H)-on-6-yl]-1,2,3,4-tetrahydronaphthalene.
m.p.: 197 to 198 °C.

(8) To 9.5 g of the above compound suspended in 100 ml of chloroform was added 6.7 ml of iodotrimethylsilane, and the mixture was refluxed under heating for 1 hour while stirring. After cooling, to the mixture was added 3 ml of methanol, and the mixture was stirred for 20 minutes. Crystals precipitated were collected by filtration to obtain 11.5 g of 2-amino-6-[4,5-dihydropyridazin-3(2H)-on-6-yl]-1,2,3,4-tetrahydronaphthalene·hydroiodide.
m.p.: 228 to 230 °C.

<u>Reference example 2</u>

(1) 2-Methoxycarbonylamino-6- (3-methoxycarbonylpropionyl)7-chloro-1,2,3,4-tetrahydronaphthalene and hydrazine monohydrate were treated in the same manner as in Reference example 1-(7) to obtain 2-methoxycarbonylamino-6-[4,5-dihydropyridazin-3(2H)-on-6-yl]-7-chloro-1,2,3,4-tetrahydronaphthalene.
m.p.: 196 to 198 °C.

(2) The above compound and iodotrimethylsilane were treated in the same manner as in Reference example 1-(8) to obtain 11.5 g of 2-amino-6-[4,5-dihydropyridazin-3(2H)-on-6-yl]-7-chloro-1,2,3,4-tetrahydronaphthalene·hydroiodide.
m.p.: >250 °C.

The desired pyridazinone derivatives (I) of the present invention and pharmaceutically acceptable salts thereof have excellent actions of protecting from endotoxin shock and excellent actions of curing nephritis so that they are useful as, for example, an agent for curing endotoxin shock which occurs in a patient seriously infected with gram-negative bacteria or an agent for curing nephritis. Further, the desired compound (I) of the present invention has low toxicity so that it can be a medicine having high safety.

## Claims

1. A pyridazinone compound represented by the formula (I):

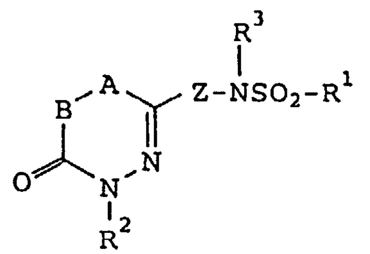

(I)

wherein

$R^1$ represents an alkyl group having 1 to 5 carbon atoms or an alkenyl group having 2 to 7 carbon atoms; $R^3$ represents hydrogen atom or an alkyl group having 1 to 6 carbon atoms; Z represents a group represented by the formula:

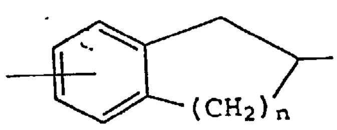

where n represents 1 or 2;
$R^2$ represents hydrogen atom, a carboxyl $C_{1-6}$ alkyl group, a tetrazolyl $C_{1-6}$ alkyl group or an alkyl group having 1 to 6 carbon atoms; and -A-B- represents ethylene group or vinylene group,

or a pharmaceutically acceptable salt thereof.

2. A compound according to claim 1 which is 2-(N-vinylsulfonyl-N-propyl)amino-5-[pyridazin-3(2H)-on-6-yl]indane or a pharmaceutically acceptable salt thereof.

3. A process for preparing a pyridazinone compound represented by the formula (I):

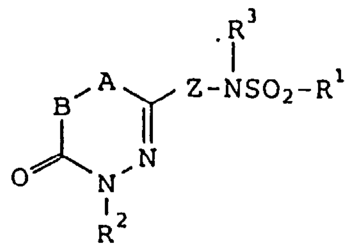

(I)

wherein

$R^1$ represents an alkyl group having 1 to 5 carbon atoms or an alkenyl group having 2 to 7 carbon atoms; $R^3$ represents hydrogen atom or an alkyl group having 1 to 6 carbon atoms; Z represents a group represented by the formula:

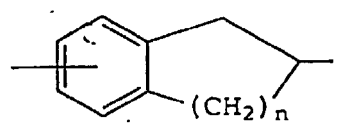

where n represents 1 or 2;
$R^2$ represents hydrogen atom, a carboxyl $C_{1-6}$ alkyl group, a tetrazolyl $C_{1-6}$ alkyl group or an alkyl group having 1 to 6 carbon atoms; and -A-B- represents ethylene group or vinylene group,

or a pharamceutically acceptable salt thereof, which comprises reacting a compound represented by the formula (II) :

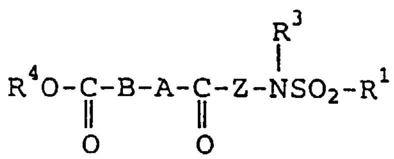

(II)

wherein $R^4$ represents hydrogen atom or an alkyl group having 1 to 6 carbon atoms; and $R^1$ , $R^3$ , Z and -A-B- have the same meanings as defined above, or a salt thereof, with a compound represented by the formula (III):

$$R^2NH\text{-}NH_2 \quad \text{(III)}$$

wherein $R^2$ has the same meaning as defined above and, if necessary, converting the resulting compound into a pharmaceutically acceptable salt thereof.

**4.** A process for preparing a pyridazinone compound represented by the formula (I-c):

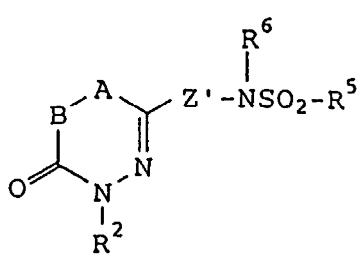

(I-c)

wherein

Z' represents a group represented by the formula:

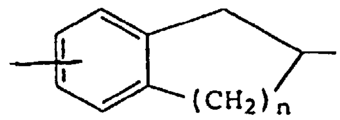

where n represents 1 to 2,
$R^5$ represents an alkyl group having 1 to 5 carbon atoms or an alkenyl group having 2 to 7 carbon atoms; $R^6$ represents hydrogen atom or an alkyl group having 1 to 6 carbon atoms; and $R^2$ represents hydrogen atom; a carboxyl $C_{1-6}$ alkyl group, a tetrazolyl $C_{1-6}$ alkyl group or an alkyl group having 1 to 6 carbon atoms; and -A-B- represents an ethylene group or a vinylene group;

or a pharmaceutically acceptable salt thereof, which comprises reacting a compound represented by the formula (IV):

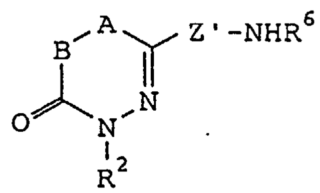

(IV)

wherein $R^6$ and Z' have the same meanings as defined above; and $R^2$ and -A-B- have the same meanings as defined above,
or a salt thereof, with a compound represented by the formula (V):

$$R^{51}\text{-}SO_2\text{-}X^1 \quad (V)$$

wherein $X^1$ represents a reactive residue; and $R^{51}$ represents a substituted or unsubstituted alkyl group having 1 to 5 carbon atoms or an alkenyl group having 2 to 7 carbon atoms,
and, if necessary, converting the resulting compound into a pharmaceutically acceptable salt thereof.

**5.** A process for preparing a pyridazinone compound represented by the formula (I-e):

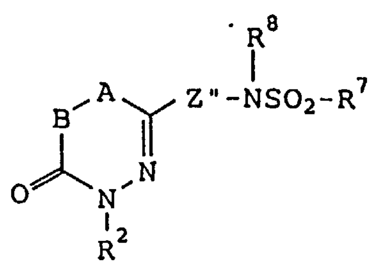

(I-e)

wherein

$R^7$ represents an alkyl group having 1 to 5 carbon atoms or an alkenyl group having 2 to 7 carbon atoms; and Z" represents a group represented by the formula:

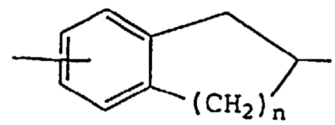

where n represents 1 or 2;
$R^8$ represents an alkyl group having 1 to 6 carbon atoms; and $R^2$ represents a hydrogen atom, a carboxyl $C_{1-6}$ alkyl group, a tetrazolyl -$C_{1-6}$ alkyl group or an alkyl group having 1 to 6 carbon atoms; and -A-B- represents an ethylene group or a vinylene group; or a pharmaceutically acceptable salt thereof, which comprises reacting a compound represented by the formula (I-d):

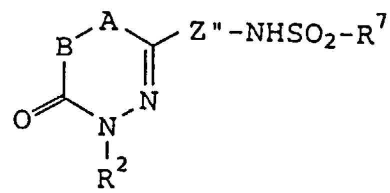

(I-d)

wherein $R^7$ and Z" have the same meanings as defined above; and $R^2$ and -A-B- have the same meanings as defined above,

or a salt thereof, with a compound represented by the formula (VI):

$$R^8\text{-}X^2 \qquad \text{(VI)}$$

wherein $X^2$ represents a reactive residue and $R^8$ has the same meaning as defined above, and, if necessary, converting the resulting compound into a pharmaceutically acceptable salt thereof.

**6.** A process for preparing a pyridazinone compound represented by the formula (I-g):

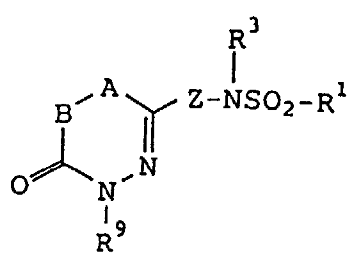

(I-g)

wherein $R^9$ represents a carboxyl $C_{1-6}$ alkyl group, a tetrazolyl $C_{1-6}$ alkyl group or an alkyl group having 1 to 6 carbon atoms; and $R^1$ represents an alkyl group having 1 to 5 carbon atoms or an alkenyl group having 2 to 7 carbon atoms; $R^3$ represents hydrogen atom or an alkyl group having 1 to 6 carbon atoms; Z represents a group represented by the formula:

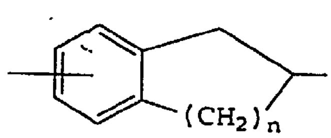

where n represents 1 or 2; and -A-B- represents ethylene group or vinylene group, or a pharmaceutically acceptable salt thereof, which comprises reacting a compound represented by the formula (I-f):

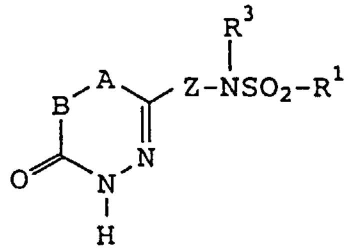

(I-f)

wherein $R^1$, $R^3$, Z and -A-B- have the same meanings as defined above, or a salt thereof, with a compound represented by the formula (VII):

$$R^9\text{-}X^3 \quad \text{(VII)}$$

wherein $X^3$ represents a reactive residue; and $R^9$ has the same meaning as defined above, and if necessary, converting the resulting compound into a pharmaceutically acceptable salt thereof.

**7.** A process for preparing a pyridazinone compound represented by the formula (I-i):

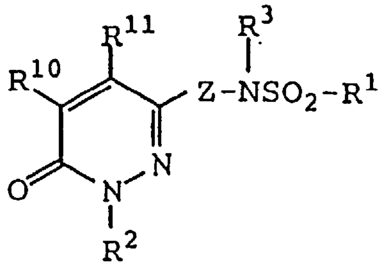

(I-i)

wherein

$R^{10}$ and $R^{11}$ represent hydrogen atom, and $R^1$ represents an alkyl group having 1 to 5 carbon atoms or an alkenyl group having 2 to 7 carbon atoms; $R^3$ represents hydrogen atom or an alkyl group having 1 to 6 carbon atoms; Z represents a group represented by the formula:

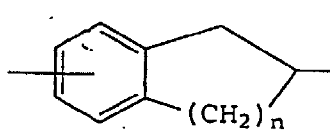

where n represents 1 or 2;
$R^2$ represents hydrogen atom, a carboxyl $C_{1-6}$ alkyl group, a tetrazolyl $C_{1-6}$ alkyl group or an alkyl group having 1 to 6 carbon atoms; and -A-B- represents ethylene group or vinylene group,

or a pharmaceutically acceptable salt thereof, which comprises oxidizing a compound represented by the formula (I-h):

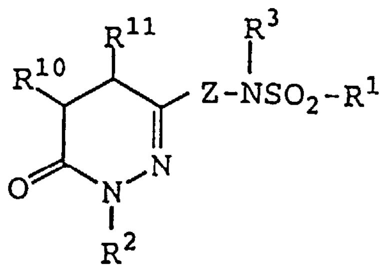

(I-h)

wherein $R^1$, $R^2$ $R^3$, $R^{10}$ and $R^{11}$ and Z have the same meanings as defined above, or a salt thereof, and, if necessary, converting the resulting compound into a pharmaceutically acceptable salt thereof.

8. A pharmaceutical composition which comprises a therapeutically effective amount of the compound as set forth in Claim 1 in admixture with a conventional pharmaceutically acceptable carrier or diluent.

9. Use of the compound or a pharmaceutically acceptable salt thereof as set forth in any one of the patent claims 1 to 2 for preparing a medicament for prophylaxis or curing of endotoxin shock and /or of nephritis.

**Patentansprüche**

1. Pyridazinon-Verbindung mit der Formel (I) :

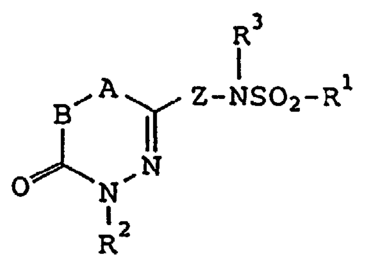

(I)

worin:

$R^1$ eine Alkyl-Gruppe mit 1 bis 5 Kohlenstoffatomen oder eine Alkenyl-Gruppe mit 2 bis 7 Kohlenstoffatomen bedeutet; $R^3$ ein Wasserstoffatom oder eine Alkyl-Gruppe mit 1 bis 6 Kohlenstoffatomen bedeutet; Z eine Gruppe mit der Formel:

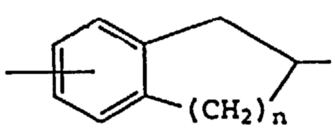

bedeutet, worin n 1 oder 2 darstellt;

$R^2$ ein Wasserstoffatom, eine Carboxyl-$C_{1-6}$-alkyl-Gruppe, eine Tetrazolyl-$C_{1-6}$-alkyl-Gruppe oder eine Alkyl-Gruppe mit 1 bis 6 Kohlenstoffatomen bedeutet; und -A-B- eine Ethylen-Gruppe oder eine Vinylen-Gruppe darstellt,

oder ein pharmazeutisch akzeptables Salz davon.

2. Verbindung gemäß Anspruch 1, die 2-(N-Vinylsulfonyl-N-propyl)amino-5-[pyridazin-3(2H)-on-6-yl]indan oder ein pharmazeutisch akzeptables Salz davon ist.

3. Verfahren zur Herstellung einer Pyridazinon-Verbindung mit der Formel (I) :

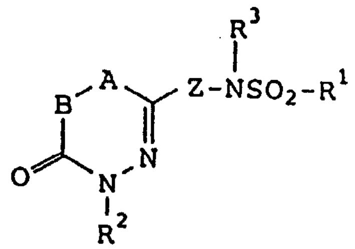

(I)

worin:

$R^1$ eine Alkyl-Gruppe mit 1 bis 5 Kohlenstoffatomen oder eine Alkenyl-Gruppe mit 2 bis 7 Kohlenstoffatomen bedeutet; $R^3$ ein Wasserstoffatom oder eine Alkyl-Gruppe mit 1 bis 6 Kohlenstoffatomen bedeutet; Z eine Gruppe mit der Formel:

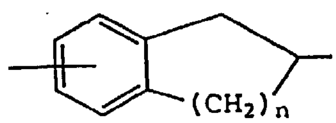

worin n 1 oder 2 bedeutet, darstellt;

$R^2$ ein Wasserstoffatom, eine Carboxyl-$C_{1-6}$-alkyl-Gruppe, eine Tetrazolyl-$C_{1-6}$-alkyl-Gruppe oder eine Alkyl-Gruppe mit 1 bis 6 Kohlenstoffatomen bedeutet; und -A-B- eine Ethylen-Gruppe oder Vinylen-Gruppe darstellt; oder ein pharmazeutisch akzeptables Salz davon, umfassend die Umsetzung einer Verbindung mti der Formel (II) :

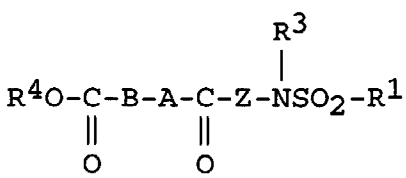

(II)

worin $R^4$ ein Wasserstoffatom oder eine Alkyl-Gruppe mit 1 bis 6 Kohlenstoffatomen darstellt; und $R^1$, $R^3$, Z und -A-B- die gleichen Bedeutungen wie oben haben, oder eines Salzes davon, mit einer Verbindung der Formel (III):

$$R^2NH\text{-}NH_2 \qquad \text{(III)}$$

worin $R^2$ die gleiche Bedeutung wie oben hat, und erforderlichenfalls Umwandlung der resultierenden Verbindung in eines ihrer pharmazeutisch akzeptablen Salze.

4. Verfahren zur Herstellung einer Pyridazinon-Verbindung mit der Formel (I-c):

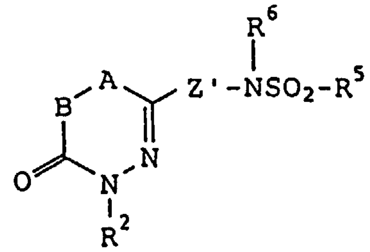

(I-c)

worin

Z' eine Gruppe der Formel:

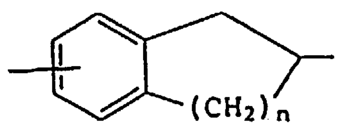

bedeutet, wobei n 1 oder 2 darstellt,

$R^5$ eine Alkyl-Gruppe mit 1 bis 5 Kohlenstoffatomen oder eine Alkenyl-Gruppe mit 2 bis 7 Kohlenstoffatomen bedeutet;

$R^6$ ein Wasserstoffatom oder eine Alkyl-Gruppe mit 1 bis 6 Kohlenstoffatomen darstellt; und $R^2$ ein Wasserstoffatom; eine Carboxyl-$C_{1-6}$-alkyl-Gruppe, eine Tetrazolyl-$C_{1-6}$-alkyl-Gruppe oder eine Alkyl-Gruppe mit 1 bis 6 Kohlenstoffatomen darstellt; und -A-B- eine Ethylen-Gruppe oder eine Vinylen-Gruppe bedeutet;

oder eines pharmazeutisch akzeptablen Salzes davon, umfassend die Umsetzung einer Verbindung mit der Formel (IV) :

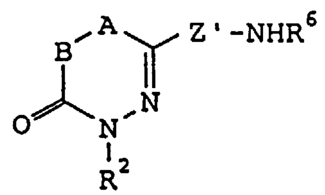

(IV)

worin $R^6$ und Z' die gleichen Bedeutungen wie oben haben und $R^2$ und -A-B- die gleichen Bedeutungen wie oben haben,
oder eines Salzes davon, mit einer Verbindung der Formel (V):

$$R^{51}\text{-}SO_2\text{-}X^1 \qquad (V)$$

worin $X^1$ einen reaktiven Rest bedeutet und $R^{51}$ eine substituierte oder unsubstituierte Alkyl-Gruppe mit 1 bis 5 Kohlenstoffatomen oder eine Alkenyl-Gruppe mit 2 bis 7 Kohlenstoffatomen darstellt,
und, falls erforderlich, Umwandlung der resultierenden Verbindung in eines ihrer pharmazeutisch akzeptablen Salze.

**5.** Verfahren zur Herstellung einer Pyridazinon-Verbindung mit der Formel (I-e):

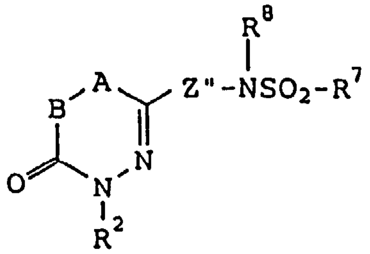

(I-e)

worin:

$R^7$ eine Alkyl-Gruppe mit 1 bis 5 Kohlenstoffatomen oder eine Alkenyl-Gruppe mit 2 bis 7 Kohlenstoffatomen darstellt; und Z" eine Gruppe mit der Formel:

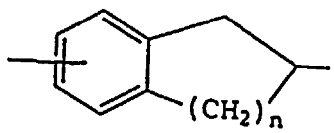

wobei n 1 oder 2 bedeutet, darstellt;

$R^8$ eine Alkyl-Gruppe mit 1 bis 6 Kohlenstoffatomen bedeutet und $R^2$ ein Wasserstoffatom, eine Carboxyl-$C_{1-6}$-alkyl-Gruppe, eine Tetrazolyl-$C_{1-6}$-alkyl-Gruppe oder eine Alkyl-Gruppe mit 1 bis 6 Kohlenstoffatomen darstellt; und -A-B- eine Ethylen-Gruppe oder eine Vinylen-Gruppe bedeutet; oder eines pharmazeutisch akzeptablen Salzes davon, umfassend die Umsetzung einer Verbindung mit der Formel (I-d):

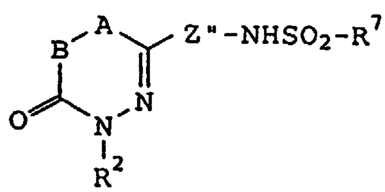

(I-d)

worin $R^7$ und Z" die gleichen Bedeutungen wie oben haben und $R^2$ und -A-B- die gleichen Bedeutungen wie oben haben,

oder eines Salzes davon, mit einer Verbindung der Formel (VI):

$$R^8\text{-}X^2 \qquad \text{(VI)}$$

worin $X^2$ einen reaktiven Rest bedeutet und $R^8$ die gleiche Bedeutung wie oben hat, und, falls erforderlich, Umwandlung der resultierenden Verbindung in eines ihrer pharmazeutisch akzeptablen Salze.

**6.** Verfahren zur Herstellung einer Pyridazinon-Verbindung mit der Formel (I-g):

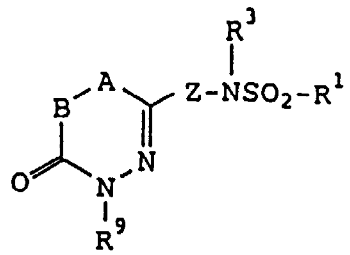

(I-g)

worin $R^9$ eine Carboxyl-$C_{1-6}$-alkyl-Gruppe, eine Tetrazolyl-$C_{1-6}$-alkyl-Gruppe oder eine Alkyl-Gruppe mit 1 bis 6 Kohlenstoffatomen bedeutet; und $R^1$ eine Alkyl-Gruppe mit 1 bis 5 Kohlenstoffatomen oder eine Alkenyl-Gruppe mit 2 bis 7 Kohlenstoffatomen bedeutet; $R^3$ ein Wasserstoffatom oder eine Alkyl-Gruppe mit 1 bis 6 Kohlenstoffatomen bedeutet; und Z eine Gruppe mit der Formel:

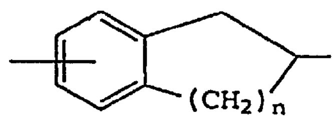

wobei n 1 oder 2 darstellt, bedeutet; und -A-B- eine Ethylen-Gruppe oder Vinylen-Gruppe bedeutet, oder eines pharmazeutisch akzeptablen Salzes davon, umfassend die Umsetzung einer Verbindung mit der Formel (I-f):

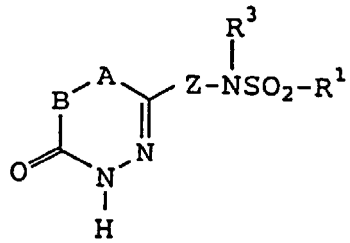

(I-f)

worin $R^1$, $R^3$, Z und -A-B- die gleichen Bedeutungen wie oben haben, oder eines Salzes davon, mit einer Verbindung der Formel (VII):

$$R^9\text{-}X^3 \qquad \text{(VII)}$$

worin $X^3$ einen reaktiven Rest bedeutet und $R^9$ die gleiche Bedeutung wie oben hat und erforderlichenfalls Umwandlung der resultierenden Verbindung in eines ihrer pharmazeutisch akzeptablen Salze.

**7.** Verfahren zur Herstellung einer Pyridazinon-Verbindung mit der Formel (I-i):

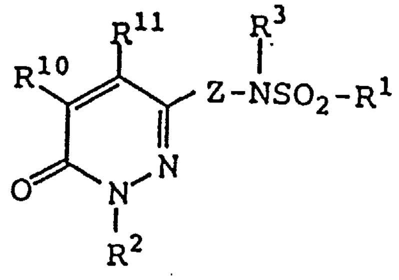

(I-i)

worin

$R^{10}$ und $R^{11}$ Wasserstoffatome bedeuten und $R^1$ eine Alkyl-Gruppe mit 1 bis 5 Kohlenstoffatomen oder eine Alkenyl-Gruppe mit 2 bis 7 Kohlenstoffatomen bedeutet; $R^3$ ein Wasserstoffatom oder eine Alkyl-Gruppe mit 1 bis 6 Kohlenstoffatomen darstellt; Z eine Gruppe mit der Formel:

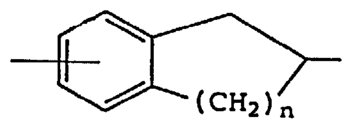

darstellt, wobei n 1 oder 2 bedeutet;

$R^2$ ein Wasserstoffatom, eine Carboxyl-$C_{1-6}$-alkyl-Gruppe, eine Tetrazolyl-$C_{1-6}$-alkyl-Gruppe oder eine Alkyl-Gruppe mit 1 bis 6 Kohlenstoffatomen bedeutet; und -A-B- eine Ethylen-Gruppe oder Vinylen-Gruppe darstellt,

oder eines pharmazeutisch akzeptablen Salzes davon, umfassend die Oxidation einer Verbindung mit der Formel (I-h):

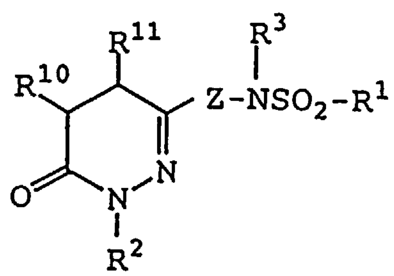

(I-h)

worin $R^1$, $R^2$ $R^3$, $R^{10}$ und $R^{11}$ und Z die gleichen Bedeutungen wie oben haben, oder eines Salzes davon, und erforderlichenfalls Umwandlung der resultierenden Verbindung in eines ihrer pharmazeutisch akzeptablen Salze.

**8.** Pharmazeutische Zusammensetzung, umfassend eine therapeutisch wirksame Menge der Verbindung gemäß Anspruch 1 in Mischung mit einem herkömmlichen pharmazeutisch akzeptablen Träger oder Verdünnungsmittel.

**9.** Verwendung der Verbindung gemäß einem der Patentansprüche 1 bis 2 oder eines pharmazeutisch akzeptablen Salzes davon zur Herstellung eines Medikaments zur Prophylaxe oder Heilung von Endotoxin-Schock und/oder Nephritis.

**Revendications**

**1.** Composé pyridazinone représenté par la formule (I) :

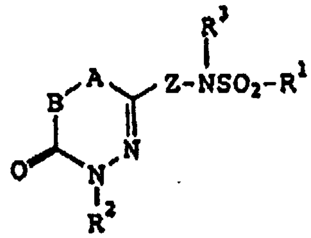

(I)

dans laquelle

$R^1$ représente un groupe alkyle ayant 1 à 5 atomes de carbone ou un groupe alcényle ayant 2 à 7 atomes de carbone ; $R^3$ représente un atome d'hydrogène ou un groupe alkyle ayant 1 à 6 atomes de carbone ; Z représente un groupe représenté par la formule :

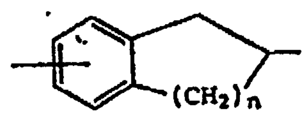

où n vaut 1 ou 2 ;
$R^2$ représente un atome d'hydrogène, un groupe carboxyl(alkyle en $C_{1-6}$), un groupe tétrazolyl(alkyle en $C_{1-6}$) ou un groupe alkyle ayant 1 à 6 atomes de carbone ; et -A-B représente un groupe éthylène ou un groupe vinylidène,

ou l'un de ses sels pharmaceutiquement acceptables.

**2.** Composé selon la revendication 1 qui est le 2-(N-vinylsulfonyl-N-propyl)amino-5-[pyridazin-3(2H)-on-6-yl]indane ou l'un de ses sels pharmaceutiquement acceptables.

**3.** Procédé de préparation d'un composé pyridazinone représenté par la formule (I) :

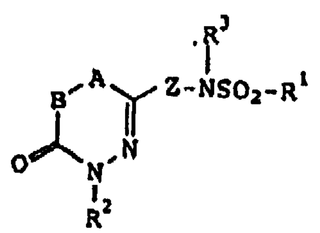

(I)

dans laquelle

$R^1$ représente un groupe alkyle ayant 1 à 5 atomes de carbone ou un groupe alcényle ayant 2 à 7 atomes de carbone ; $R^3$ représente un atome d'hydrogène ou un groupe alkyle ayant 1 à 6 atomes de carbone ; Z représente un groupe représenté par la formule :

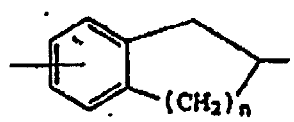

où n vaut 1 ou 2 ;
$R^2$ représente un atome d'hydrogène, un groupe carboxyl(alkyle en $C_{1-6}$), un groupe tétrazolyl(alkyle en $C_{1-6}$) ou un groupe alkyle ayant 1 à 6 atomes de carbone ; et -A-B représente un groupe éthylène ou un groupe vinylidène,

ou l'un de ses sels pharmaceutiquement acceptables qui comprend la réaction d'un composé représenté par la formule (II) :

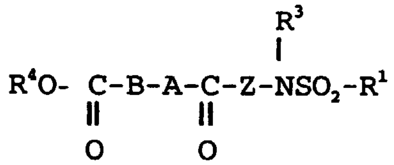

(II)

dans laquelle $R^4$ représente un atome d'hydrogène ou un groupe alkyle ayant 1 à 6 atomes de carbone ; et $R^1$, $R^3$, Z et -A-B ont les mêmes significations que celles définies ci-dessus, ou de l'un de ses sels, avec un composé représenté par la formule (III) :

$$R^2NH\text{-}NH_2 \quad \text{(III)}$$

dans laquelle $R^2$ a la même signification que celle définie ci-dessus et, si nécessaire, la conversion du composé obtenu en l'un de ses sels pharmaceutiquement acceptables.

**4.** Procédé de préparation d'un composé pyridazinone représenté par la formule (I-c) :

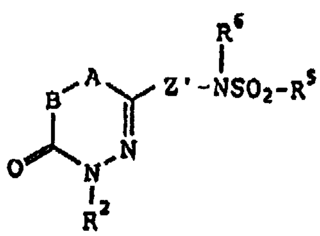

(I-c)

dans laquelle

Z' représente un groupe représenté par la formule :

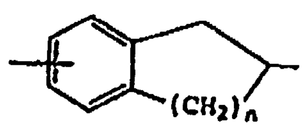

où n vaut 1 ou 2,
$R^5$ représente un groupe alkyle ayant 1 à 5 atomes de carbone ou un groupe alcényle ayant 2 à 7 atomes de carbone ; $R^6$ représente un atome d'hydrogène ou un groupe alkyle ayant 1 à 6 atomes de carbone ;

et $R^2$ représente un atome d'hydrogène ; un groupe carboxyl(alkyle en $C_{1\text{-}6}$), un groupe tétrazolyl(alkyle en $C_{1\text{-}6}$) ou un groupe alkyle ayant 1 à 6 atomes de carbone ; et -A-B représente un groupe éthylène ou un groupe vinylidène, ou l'un de ses sels pharmaceutiquement acceptables, qui comprend la réaction d'un composé représenté par la formule (IV) :

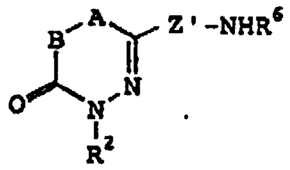

(IV)

dans laquelle $R^6$ et Z' ont les mêmes significations que celles définies ci-dessus ; et $R^2$ et -A-B- ont les mêmes significations que celles définies ci-dessus
ou de l'un de ses sels, avec un composé représenté par la formule (V) :

$$R^{51}\text{-}SO_2\text{-}X^1 \qquad (V)$$

dans laquelle $X^1$ représente un résidu actif ; et $R^{51}$ représente un groupe alkyle substitué ou non substitué ayant 1 à 5 atomes de carbone ou un groupe alcényle ayant 2 à 7 atomes de carbone,
et, si nécessaire, la conversion du composé obtenu en l'un de ses sels pharmaceutiquement acceptables.

**5.** Procédé de préparation d'un composé pyridazinone représenté par la formule (I-e) :

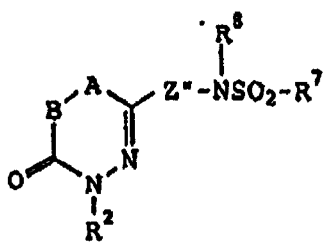

(I-e)

dans laquelle

$R^7$ représente un groupe alkyle ayant 1 à 5 atomes de carbone ou un groupe alcényle ayant 2 à 7 atomes de carbone ; et Z" représente un groupe représenté par la formule :

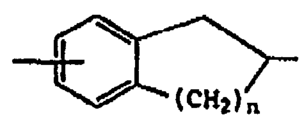

où n vaut 1 ou 2,
$R^8$ représente un groupe alkyle ayant 1 à 6 atomes de carbone ; et $R^2$ représente un atome d'hydrogène ; un groupe carboxyl(alkyle en $C_{1-6}$), un groupe tétrazolyl(alkyle en $C_{1-6}$) ou un groupe alkyle ayant 1 à 6 atomes de carbone ; et -A-B représente un groupe éthylène ou un groupe vinylidène, ou l'un de ses sels pharmaceutiquement acceptables, qui comprend la réaction d'un composé représenté par la formule (I-d):

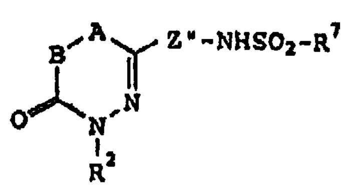

(I-d)

dans laquelle $R^7$ et Z" ont les mêmes significations que celles définies ci-dessus ; et $R^2$ et -A-B- ont les mêmes

significations que celles définies ci-dessus

ou l'un de ses sels, avec un composé représenté par la formule (VI):

$$R^8\text{-}X^2 \qquad \text{(VI)}$$

dans laquelle $X^2$ représente un résidu actif et $R^8$ a la même signification que celle définie ci-dessus, et, si nécessaire, la conversion du composé obtenu en l'un de ses sels pharmaceutiquement acceptables.

**6.** Procédé de préparation d'un composé pyridazinone représenté par la formule (I-g):

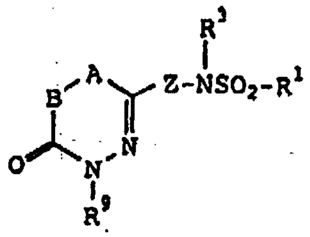

(I-g)

dans laquelle $R^9$ représente un groupe carboxyle en $C_{1-6}$, un groupe tétrazolyl(alkyle en $C_{1-6}$) ou un groupe alkyle ayant 1 à 6 atomes de carbone ; et $R^1$ représente un groupe alkyle ayant 1 à 5 atomes de carbone ou un groupe alcényle ayant 2 à 7 atomes de carbone ; $R^3$ représente un atome d'hydrogène ou un groupe alkyle ayant 1 à 6 atomes de carbone ; Z représente un groupe représenté par la formule :

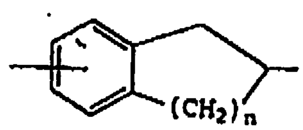

où n vaut 1 ou 2 ; et -A-B représente un groupe éthylène ou un groupe vinylidène,
ou l'un de ses sels pharmaceutiquement acceptables qui comprend la réaction d'un composé représenté par la formule (I-f) :

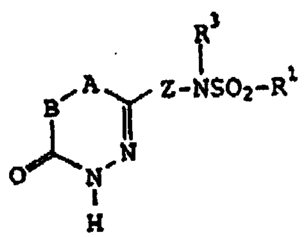

(I-f)

dans laquelle $R^1$, $R^3$, Z et -A-B ont les mêmes significations que celles définies ci-dessus ;
ou l'un de ses sels, avec un composé représenté par la formule (VII) :

$$R^9\text{-}X^3 \qquad \text{(VII)}$$

dans laquelle $X^3$ représente un résidu actif ; et $R^9$ a la même signification que définie ci-dessus, et, si nécessaire, la conversion du composé obtenu en l'un de ses sels pharmaceutiquement acceptables.

**7.** Procédé de préparation d'un composé pyridazinone représenté par la formule (I-i) :

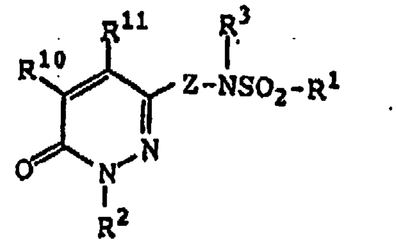

(I-i)

dans laquelle

$R^{10}$ et $R^{11}$ représentent un atome d'hydrogène, et $R^1$ représente un groupe alkyle ayant 1 à 5 atomes de carbone ou un groupe alcényle ayant 2 à 7 atomes de carbone ; $R^3$ représente un atome d'hydrogène ou un groupe alkyle ayant 1 à 6 atomes de carbone ; Z représente un groupe représenté par la formule :

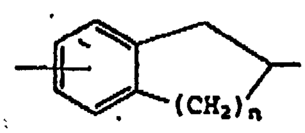

où n vaut 1 ou 2 ;
$R^2$ représente un atome d'hydrogène, un groupe carboxyl(alkyle en $C_{1-6}$), un groupe tétrazolyl(alkyle en $C_{1-6}$) ou un groupe alkyle ayant 1 à 6 atomes de carbone ; et -A-B représente un groupe éthylène ou un groupe vinylidène,

ou l'un de ses sels pharmaceutiquement acceptables, qui comprend l'oxydation d'un composé représenté par la formule (I-h) :

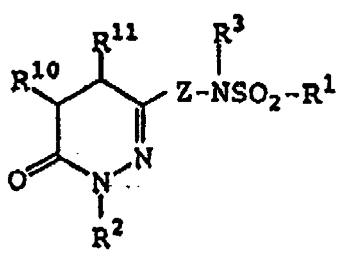

. (I-h)

dans laquelle $R^1$, $R^2$, $R^3$, $R^{10}$, $R^{11}$ et Z ont les mêmes significations que celles définies ci-dessus ;
ou de l'un de ses sels, et, si nécessaire, la conversion du composé obtenu en l'un de ses sels pharmaceutiquement acceptables.

**8.** Composition pharmaceutique qui comprend une quantité thérapeutiquement efficace du composé tel que décrit dans la revendication 1 en mélange avec un véhicule ou un diluant pharmaceutiquement acceptable conventionnel.

**9.** Utilisation du composé ou de l'un de ses sels pharmaceutiquement acceptable tels que décrits dans les revendications du brevet 1 à 2, pour la préparation d'un médicament pour la prophylaxie ou le traitement d'un choc endotoxinique et/ou de néphrite.